(19) Europäisches Patentamt
European Patent Office
Office européen des brevets

(11) **EP 1 680 025 B1**

(12) **FASCICULE DE BREVET EUROPEEN**

(45) Date de publication et mention
de la délivrance du brevet:
**12.07.2017 Bulletin 2017/28**

(21) Numéro de dépôt: **04791537.6**

(22) Date de dépôt: **14.10.2004**

(51) Int Cl.:
*A61B 5/00* *(2006.01)*     *A61B 8/08* *(2006.01)*

(86) Numéro de dépôt international:
**PCT/FR2004/002625**

(87) Numéro de publication internationale:
**WO 2005/041779 (12.05.2005 Gazette 2005/19)**

(54) **PROCEDE ET SYSTEME DE DETERMINATION DE LA MASSE GRASSE CORPORELLE TOTALE, PROCEDE ET SYSTEME DE DETERMINATION DE LA COMPOSITION CORPORELLE**

VERFAHREN UND SYSTEM ZUR BESTIMMUNG DER GESAMTMENGE AN KÖRPERFETT, VERFAHREN UND SYSTEM ZUR BESTIMMUNG DER KÖRPERZUSAMMENSETZUNG

METHOD AND SYSTEM FOR DETERMINING THE TOTAL AMOUNT OF BODY FAT, METHOD AND SYSTEM FOR DETERMINING BODY COMPOSITION

(84) Etats contractants désignés:
**AT BE BG CH CY CZ DE DK EE ES FI FR GB GR HU IE IT LI LU MC NL PL PT RO SE SI SK TR**

(30) Priorité: **20.10.2003 FR 0312234**

(43) Date de publication de la demande:
**19.07.2006 Bulletin 2006/29**

(73) Titulaire: **CENTRE NATIONAL DE LA RECHERCHE SCIENTIFIQUE -CNRS- 75794 Paris Cedex 16 (FR)**

(72) Inventeurs:
• **Pineau, Jean-Claude**
**F-75005 Paris (FR)**
• **BOCQUET, Michel**
**F-95800 Cergy Saint Christophe (FR)**
• **Crescenzo, Eric**
**F-71590 Gergy (FR)**

(74) Mandataire: **Regimbeau**
**20, rue de Chazelles**
**75847 Paris Cedex 17 (FR)**

(56) Documents cités:
**EP-A- 1 055 396     US-A- 5 941 825**

• **TO W W K ET AL: "ASSOCIATION BETWEEN BODY COMPOSITION AND MENSTRUAL DYSFUNCTION IN COLLEGIATE DANCE STUDENTS" JOURNAL OF OBSTETRICS AND GYNAECOLOGY RESEARCH, XX, JP, vol. 23, no. 6, 1997, pages 529-535, XP008031321 ISSN: 1341-8076**
• **FRIEDL ET AL.: "Evaluation of antropometric equations to assess body-composition changes in young women" AMERICAN JOURNAL OF CLINICAL NUTRITION, vol. 73, no. 2, février 2001 (2001-02), pages 268-275, XP002286560 us**
• **WONG ET AL.: "Body composition of lactating women determined by anthropometry and deuterium dilution" BRITISH JOURNAL OF NUTRITION, vol. 61, 1989, pages 25-33, XP008032171 GB**

**Description**

La présente invention concerne le domaine des techniques de détermination de la masse grasse totale dans le corps d'un être humain. La masse totale d'un individu est répartie en la masse maigre d'une part, qui se compose des muscles, du contenu minéral et de l'eau totale, et en la masse grasse d'autre part. La composition corporelle d'un individu est définie par les valeurs de masse grasse, masse maigre et eau corporelle totale (intracellulaire et extracellulaire) présentes dans son corps. Sous certaines conditions, elle peut être déterminée à partir de la masse grasse.

De nombreuses disciplines utilisent les valeurs de masse grasse totale, ou les valeurs de composition corporelle, d'individus. Parmi ces disciplines, on peut citer par exemple l'épidémiologie, qui peut rechercher les divers facteurs relatifs aux valeurs de masse grasse ou de composition corporelle d'un échantillon représentatif de groupes d'individus, conditionnant l'apparition, la fréquence, le mode de diffusion et l'évolution de maladies affectant ces groupes d'individus.

Par ailleurs ces notions de masse grasse ou de composition corporelle peuvent également être utilisées dans le cadre de la mise en place ou du suivi de régimes alimentaires spécifiques, ou de remodelage du corps, dans un contexte grand public (services dispensés sur réseau internet ou dans des instituts d'esthétique), paramédical ou médical. Des équipes de praticiens sont notamment spécialisées dans le suivi de sportifs de haut niveau.

Il existe différentes techniques pour déterminer la masse grasse corporelle totale d'un individu. Certaines de ces techniques sont analysées dans le document de HC Lukaski « Methods for the assessment of human body composition : traditional and new » (Am J clin Nutr 1987 ; 46 :537-56). La mesure par absorptiométrie biphotonique, encore appelée DEXA ("Dual Energy X Ray Absorptiometry") fournit des résultats très fiables et est une méthode de référence de la masse grasse totale corporelle et de la composition corporelle. Elle consiste en l'irradiation de l'individu par un faisceau de photons à deux énergies et permet de distinguer l'absorption des différents tissus et de calculer leur masse. Toutefois, elle présente l'inconvénient de requérir l'irradiation de l'individu concerné par la mesure, par une faible dose de rayons X et de ne pouvoir être mise en oeuvre qu'en milieu hospitalier.

Par ailleurs, l'imagerie par résonance médicale ou IRM permet de déterminer la masse grasse en autant de sections de l'individu que de plans de coupes pratiqués lors de l'examen. Il est ensuite possible à partir de ces mesures sur les plans de coupe d'estimer la masse grasse corporelle totale. Cette technique est par exemple décrite dans le document de Han TS, Kelly IE, Walsh K, Greene RME, Lean MEJ, « Relationship between volumes and areas from single transverse scans of intra abdominal fat measured by magnetic resonance imaging » (Int J Obes 1997; 21 :1161-1166). Cependant, le processus est long, et le matériel nécessaire est coûteux. Cette technique, ne s'exécutant qu'en milieu hospitalier, n'est pas adaptée aux examens de routine, du fait de l'ampleur des moyens mis en oeuvre.

Il existe également des techniques pouvant être mises en oeuvre en-dehors des structures hospitalières, et nécessitant un matériel moins coûteux que les méthodes DEXA ou IRM. Parmi celles-ci, on peut citer la méthode de la pince à plis cutanés, qui consiste à saisir fermement un pli cutané et à déterminer à partir de l'épaisseur du pli et d'équations mathématiques, la masse grasse. Cependant cette technique de détermination est sujette à des erreurs lors de la mesure par les opérateurs. De plus, elle n'est pas valable notamment sur des individus obèses.

Par ailleurs, le document US-A-5,941,825 « Measurement of body fat using ultrasound methods and devices » de Gramp Stephan, Lang Philipp et Mendlein John décrit une technique de détermination de la masse grasse corporelle totale d'un individu utilisant le même principe que celui exposé par Jackson et Polock en 1978 avec la méthode de la pince à plis cutanés, qui déterminaient par une formule le pourcentage de graisse totale grâce à des mesures d'épaisseurs de masse grasse au niveau des bras, de la poitrine, de l'abdomen et des cuisses. Le document US-A-5,941,825 emploie une nouvelle formule et un principe de mesure de l'épaisseur de masse grasse par ultrasons.

Selon le document US-A-5,941,825, on mesure en onze points une épaisseur de masse grasse sous-cutanée locale à l'aide d'un dispositif à ultrasons. Ces points correspondent respectivement aux zones suivantes : mollet interne, mollet latéral, cuisse antérieure, cuisse postérieure, triceps, biceps, poitrine, abdomen, aisselle, région sous-scapulaire et région supra-iliaque. Puis on déduit un volume de masse grasse à partir de ces onze valeurs d'épaisseur de masse grasse locale à partir d'une formule, et on multiplie ce volume estimé par une densité de masse grasse pour déterminer la masse grasse corporelle totale.

Cette technique présente l'avantage de pouvoir être réalisée dans des structures non médicales. Cependant, elle nécessite une étape de mesure relativement longue, puisque celle-ci comporte onze points. D'autre part, une étude comparée des résultats fournis par cette méthode et par la technique de référence DEXA met en valeur des écarts notables.

Un but de la présente invention est de pallier au moins certaines des limitations des techniques de détermination de la masse grasse corporelle totale de l'art antérieur.

Ainsi, suivant un premier objet, la présente invention propose un procédé de détermination de la masse grasse corporelle totale d'un individu, caractérisé en ce qu'il comprend les étapes suivantes :

- on mesure une épaisseur de graisse sous-cutanée en quatre points du corps de l'individu, situés respectivement au niveau antérieur droit de la mi-cuisse gauche, au niveau antérieur gauche de la mi-cuisse droite, au niveau ombilical dorsal droit et au niveau ombilical dorsal gauche ;

- on détermine ensuite une première estimation de la masse grasse corporelle totale de l'individu en fonction d'au moins les mesures d'épaisseur de graisse sous-cutanée en lesdits quatre points.

Le procédé selon l'invention permet ainsi d'obtenir une estimation de la masse grasse corporelle totale à partir de la mesure de l'épaisseur de graisse sous-cutanée en un nombre réduit de points : la détermination peut donc être effectuée rapidement. Le document EP 1 055 396 propose quant à lui une détermination de la masse graisseuse à partir de l'indice de masse corporelle, ainsi que de paramètres tels que l'âge, l'origine ethnique et le sexe. En revanche, le document EP 1 055 396 ne propose pas de réaliser des mesures spécifiques sur le corps du sujet.

Le document de Friedl K E, Westphal K A, Marchitelli L J, Patton J F, Cameron Chumlea W, and Guo S S «Evaluation of anthorpometric equations to assess body-composition changes in young women» (Ann. J. Clin. Nutr. 2001 (73): 268-275) décrit des points de mesure.

Avantageusement, les mesures d'épaisseur de masse grasse sous-cutanée locales peuvent être effectuées à l'aide d'un dispositif à ultra-sons. Ainsi le procédé peut être mis en oeuvre en toute structure et ne nécessite pas un environnement médical. De plus, il permet d'éviter le traumatisme engendré par toute exposition aux rayons X, même très courte.

Toutefois, dans un autre mode de mise en oeuvre permettant une détermination plus rapide de la masse grasse totale, l'épaisseur de graisse sous-cutanée en certains au moins de ces points peut être mesurée à l'aide d'autres moyens (pince à plis etc). Elle peut également être estimée -et non mesurée-, par exemple à l'aide d'abaques et à partir de données anthropométriques relatives à l'individu, telles que le poids, la taille, le périmètre de certaines sections de son corps, fournies par des dispositifs très usuels (balance, mètre ruban). Toutefois, la justesse du résultat obtenu sera moins grande.

Dans des modes de mise en oeuvre préférés du procédé selon l'invention, on pourra avoir recours à l'une ou l'autre des dispositions suivantes :

- la première estimation de la masse grasse corporelle totale de l'individu est en outre fonction de valeurs représentatives du périmètre du tour de taille passant par l'ombilic, du périmètre du tour de jambe passant à mi-cuisse, de l'indice de corpulence défini comme le rapport entre le poids et le carré de la stature, et du sexe de l'individu ;
- la première estimation de la masse grasse corporelle totale de l'individu est une fonction linéaire des mesures d'épaisseur de graisse sous-cutanée en lesdits quatre points ;
- la première estimation de la masse grasse corporelle totale de l'individu est obtenue à l'aide de l'équation ci-dessous :

$$MG = a\ BMI + b\ Eombg + c\ Eombd + d\ PTAI + e\ Ecuid + f\ Ecuig + g\ PCUI + Ct,$$

où MG est la première estimation de la masse grasse corporelle totale en kg, BMI est le rapport entre le poids en kg et le carré de la stature en m, PTAI est le périmètre du tour de taille passant par l'ombilic, PCUI est la moyenne des périmètres du tour de jambes gauche et droite passant à mi-cuisse, Eombg, Eombd, Ecuid et Ecuig sont les épaisseurs de graisse mesurées respectivement au niveau ombilical dorsal gauche, au niveau ombilical dorsal droit, au niveau antérieur droit de la mi-cuisse gauche et au niveau antérieur gauche de la mi-cuisse droite, en mm,
et où a = 0,959, b = 0,119, c = -0,044, d = 0,13, e = 0,586, f = -0,329, g =0,061, Ct = -23,88, lorsque l'individu est de sexe féminin ;
et a = 0,733, b = -0,053, c = 0,102, d = 0,27, e = 0,155, f = -0,106, g = 0,015, Ct = -31,21, lorsque l'individu est de sexe masculin ;

- le procédé comprend en outre une étape de comparaison entre la première estimation de la masse grasse corporelle totale de l'individu et un seuil défini par sexe, puis une étape de détermination d'une seconde estimation de la masse grasse corporelle totale de l'individu en fonction des résultats de la comparaison et d'au moins des mesures d'épaisseur de graisse sous-cutanée en lesdits quatre points ;

- la seconde estimation de la masse grasse corporelle totale de l'individu est en outre fonction au moins de valeurs représentatives du périmètre du tour de taille passant par l'ombilic, du périmètre du tour de jambe passant à mi-cuisse, de l'indice de corpulence défini comme le rapport entre le poids et le carré de la stature, et du sexe de l'individu ;

- la seconde estimation de la masse grasse corporelle totale de l'individu est obtenue à l'aide d'une fonction linéaire des mesures d'épaisseur cutanée en lesdits quatre points ;

- la seconde estimation de la masse grasse corporelle totale de l'individu est obtenue à l'aide de l'équation ci-dessous :

MG = a BMI + b Eombg + c Eombd + d PTAI + e Ecuid + f Ecuig + g PCUI + Ct, lorsque l'individu est de sexe féminin où a = 0,947, b = 0,079, c = 0,0067, d = 0,103, e = -0,303, f = 0,379, g =0,072, Ct = -20,58 si la première estimation est inférieure au seuil prédéfini de 42 kg, et a = -0,743, b = -1,73, c = 1,10, d = 1,677, e = -3,35, f = 2,62, g = -4,95, Ct = 242,53 si la première estimation est supérieure ou égale au seuil prédéfini de 42 kg, et à l'aide de l'équation suivante lorsque l'individu est de sexe masculin :

$$MG = a\ BMI + b\ Eombg + c\ Eombd + d\ PTAI + e\ Ecuid + f\ Ecuig + g\ PCUI + h\ Emcui + i\ Emomb + Ct,$$

où Emcui est l'épaisseur de graisse moyenne à mi-cuisse et Emomb est l'épaisseur de graisse moyenne au niveau dorsal
et où a = 0,221, b = 0,032, c = 0,06, d = 0,253, e = 0,078, f = -0,0128, g = 0,073, h = -0,048, i = -0,028, Ct = -21,2 si la première estimation est inférieure au seuil prédéfini de 22 kg, et a = 1,31, b = -2,69, c = -1,76, d = -0,175, e = 10,33, f = 8,91, g = -0,123, h = -18,97, i = 4,72, Ct = -10,88 si la première estimation est supérieure ou égale au seuil prédéfini de 22 kg.

Suivant un deuxième objet, l'invention propose un procédé de détermination de la composition corporelle d'un individu selon lequel on réalise les étapes d'un procédé de détermination de la masse grasse corporelle totale de individu telle que définie ci-dessus. Puis, à partir de la masse grasse déterminée, on détermine une valeur de masse maigre et une valeur d'eau corporelle totale présentes dans le corps de l'individu.
Avantageusement, la valeur de la masse maigre est égale au poids de l'individu auquel on soustrait une masse grasse déterminée et la valeur d'eau corporelle totale est égale à :

Ecorp = 0,692 MM + 1,572, où Ecorp est l'eau corporelle totale (en litre) et MM la masse maigre (en kilogramme).

Suivant un troisième et quatrième objet, l'invention propose un système de détermination de la masse grasse corporelle totale d'un individu, respectivement un système de détermination de la composition corporelle d'un individu, comprenant des moyens pour mettre en oeuvre les étapes d'un procédé selon le premier objet, respectivement le second objet de l'invention.
De tels systèmes selon l'invention sont des systèmes fiables de détermination de la masse grasse corporelle totale, respectivement de la composition corporelle totale, qui peuvent aisément être transportés d'un lieu en un autre, et être utilisés dans des applications non encadrées par du personnel médical. Cette caractéristique est intéressante notamment dans le suivi des sportifs car elle permet d'effectuer aisément le contrôle routinier de la masse grasse totale en cas de manifestation sportive itinérante.
D'autres caractéristiques et avantages de l'invention apparaîtront encore à la lecture de la description qui va suivre. Celle-ci est purement illustrative et doit être lue en regard des dessins annexés sur lesquels :

- la figure 1 est un schéma de principe d'un mode de réalisation d'un système selon l'invention ;
- les figures 2a et 2b présentent la localisation des points de mesure dans le mode de réalisation de l'invention illustré en figure 1 ;
- la figure 3a représente un individu vu de face sur lequel a été disposée une ceinture dans un mode de réalisation d'un système selon l'invention ;
- la figure 3b représente un individu vu de dos sur lequel a été disposée une ceinture dans un mode de réalisation d'un système selon l'invention ;
- la figure 4a illustre la corrélation, pour un groupe représentatif de femmes, entre les valeurs de masse grasse totale déterminées, dans un mode de mise en oeuvre de l'invention et celles déterminées par la méthode de référence DEXA ;
- la figure 4b illustre la corrélation, pour un groupe représentatif d'hommes, entre les valeurs de masse grasse totale déterminées dans un mode de mise en oeuvre de l'invention et celles déterminées par la méthode de référence DEXA.

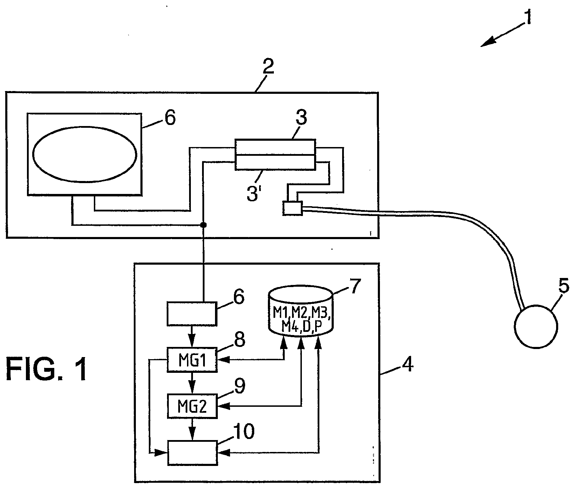

La figure 1 est un schéma de principe d'un mode de réalisation d'un système 1 selon l'invention. Ce système comprend un échographe 2. Cet échographe 2 est par exemple de représentation type-A ou uniaxial.

L'échographe 2 comporte un circuit d'attaque 3 et un circuit de réception 3' qui respectivement permettent d'effectuer des tirs ultrasonores depuis une sonde ultrasonore d'émission-réception 5, et recueillent les mesures des échos reçus par la sonde 5 suite à ces tirs. La sonde 5 connectée aux circuits 3 et 3' est par exemple une sonde droite mono-élément ou bi-élément focalisante ou non et utilisée notamment dans les applications vétérinaires pour la mesure d'épaisseur de gras de porc.

Le dispositif comprend en outre dans un mode de mise en oeuvre avantageux un calculateur 4 relié à l'échographe 2. Ce calculateur 4 comporte un module de détermination d'épaisseur de masse grasse sous-cutanée 6, adapté pour piloter le circuit d'attaque 3 et pour traiter les mesures d'échos successivement fournies par le circuit de réception 3', de manière à en extraire des valeurs représentatives d'une épaisseur de masse grasse sous-cutanée locale correspondant à la position de la sonde 5, par exemple après une phase de calibration réglant le gain et l'amplitude de l'échographe 2.

Dans un autre mode de réalisation, la correspondance entre l'amplitude des échos mesurés et l'épaisseur de graisse sous-cutanée est réalisée à l'aide d'abaques.

Le fonctionnement d'un dispositif pour déterminer par ultrasons une épaisseur de graisse sous-cutanée est bien connue de l'homme du métier (voir par exemple le document WO 99/65395).

Le système 1 comporte avantageusement une mémoire 7 pour stocker des épaisseurs de masse grasse calculées. Ce stockage peut avoir lieu de façon automatique ou sur validation de l'opérateur du système 1.

Dans le mode de mise en oeuvre représenté en figure 1, le calculateur 4 comporte également un premier module de détermination de masse grasse corporelle totale 8 calculant une première estimation de la masse grasse corporelle totale d'un individu MG1, en calculant une fonction linéaire F à partir d'un ensemble de quatre mesures d'épaisseur de graisse sous-cutanée Mombg, Mombd, Mcuid et Mcuig stockées dans la mémoire 7, et d'autres données D qui peuvent être introduites avant le calcul par l'opérateur, ou stockées elles aussi préalablement en mémoire 7.

Le calculateur 4 comporte un deuxième module de détermination de masse grasse corporelle totale 9 effectuant tout d'abord une comparaison entre la première estimation MG1 délivrée par le module 8 et un seuil prédéterminé dépendant du sexe de l'individu, Sf étant le seuil pour les femmes (Sf = 42 kg) et Sh étant le seuil pour les hommes (Sh = 22 kg) et calculant ensuite une seconde estimation de la masse grasse corporelle totale d'un individu MG2, par l'intermédiaire d'une fonction linéaire dépendant des résultats de la comparaison, à partir des quatre mesures d'épaisseur de graisse sous-cutanée Mombg, Mombd, Mcuid et Mcuig stockées dans la mémoire 7, et des données D.

Le calculateur 4 comporte un troisième module de détermination de composition corporelle totale 10, qui à partir de l'estimation de masse grasse corporelle totale MG 2 déterminée par le module 9 (ou à partir de l'estimation de masse grasse corporelle totale MG 1 déterminée par le module 8), délivre une estimation de la masse maigre et de l'eau totale figurant dans le corps de l'individu.

L'échographe 2 comporte en outre un écran 6 permettant de visualiser les mesures effectuées.

Le mode opératoire pour déterminer la masse grasse corporelle totale d'un individu est le suivant : après avoir appliqué un gel de couplage entre la sonde 5 et la peau de l'individu, un opérateur réalise une mesure d'épaisseur de graisse sous-cutanée en quatre points Mombg, Mombd, Mcuid et Mcuig du corps de l'individu en appliquant la sonde 5 successivement en chacun de ces points et en déclenchant le tir à ultrasons par le circuit 3 de l'échographe 2. Les échos reçus par la sonde 5 en chacun de ces points sont traités par le circuit 3' puis par le calculateur 6 qui délivre l'épaisseur de masse grasse sous-cutanée Eombg, Eombd, Ecuid, Ecuig en chacun des points Mombg, Mombd, Mcuid et Mcuig. Ces valeurs Eombg, Eombd, Ecuid, sont stockées en mémoire 6.

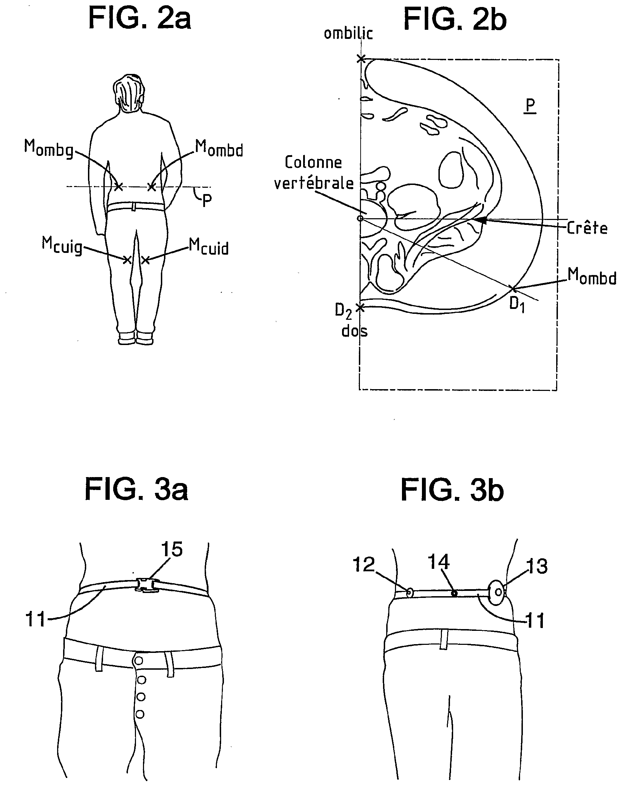

Le point Mcuig est situé au niveau antérieur droit de la mi-cuisse gauche d'un individu représenté de dos en figure 2a, le point Mcuid au niveau antérieur gauche de la mi-cuisse droite. Ils sont situés à environ 22 cm au-dessus de la rotule, au quart antérieur soit 11h30 pour la cuisse droite et 1h30 pour la cuisse gauche, en imaginant une horloge pour laquelle midi correspond à la génératrice extrême avant et 18h à la génératrice extrême arrière.

Comme représenté sur l'individu représenté de dos en figure 2a et sur une vue de cet individu en demi-coupe horizontale au niveau ombilical représentée en figure 2b, le point Mombd se trouve au niveau ombilical dorsal droit : dans le dos, dans un plan horizontal P traversant le tronc de l'individu (situé en position verticale) et passant par l'ombilic, la droite D1 du plan P passant par Mombd et la colonne vertébrale formant un angle d'environ 45 ° avec la droite D2 du plan P, passant par l'ombilic et la colonne vertébrale. Le point Mombg est disposé de façon similaire au niveau ombilical dorsal gauche. Il est ainsi disposé de façon sensiblement symétrique au point Mombd par rapport à la droite D2.

Un ensemble de données D anthropomorphiques relatives à l'individu a été préalablement stocké par l'opérateur en mémoire M7. Ces données D comportent le sexe de l'individu (féminin ou masculin), le rapport entre le poids en kg et le carré de la stature en m (BMI), le périmètre du tour de taille passant par l'ombilic (PTAI) et la moyenne des périmètres du tour des jambes gauche et droite passant à mi-cuisse (PCUI).

On calcule ensuite, à l'aide du module 8 de détermination de masse grasse corporelle totale, une première estimation en kg de la masse grasse corporelle totale de l'individu définie par la fonction linéaire suivante :

$$MG1 = a\ BMI + b\ Eombg + c\ Eombd + d\ PTAI + e\ Ecuid + f\ Ecuig + g\ PCUI + Ct, \quad (E1)$$

avec a = 0,959, b = 0,119, c = -0,044, d = 0,13, e = 0,586, f = -0,329, g =0,061, Ct = -23,88 lorsque l'individu est de sexe féminin,
et a = 0,733, b = -0,053, c = 0,102, d = 0,27, e = 0,155, f = -0,106, g = 0,015, Ct = -31,21 lorsque l'individu est de sexe masculin.

On affine ensuite ces résultats à l'aide du module 9, qui va calculer une nouvelle estimation MG2 de la masse grasse corporelle de l'individu après avoir comparé la première estimation obtenue MG1 respectivement au seuil Sf si l'individu est de sexe féminin, au seuil Sh si l'individu est de sexe masculin.
Cette seconde estimation MG2 est définie, de la façon suivante si l'individu est de sexe féminin, à partir des données D et des mesures d'épaisseur de graisse sous-cutanée utilisées pour la détermination de la première estimation :

$$MG2 = a\ BMI + b\ Eombg + c\ Eombd + d\ PTAI + e\ Ecuid + f\ Ecuig + g\ PCUI + Ct,$$

- où a = 0,947, b = 0,079, c = 0,0067, d = 0,103, e = -0,303, f = 0,379, g =0,072, Ct = -20,58 si la première estimation MG1 est inférieure au seuil Sf égal à 42 kg,
- et a = -0,743, b = -1,73, c = 1,10, d = 1,677, e = -3,35, f = 2,62, g = - 4,95, Ct = 242,53 si la première estimation MG1 est supérieure ou égale au seuil Sf égal 42 kg,

et de la façon ci-après , à partir des données D et des mesures d'épaisseur de graisse sous-cutanée utilisées pour la détermination de la première estimation si l'individu est de sexe masculin :

$$MG = a\ BMI + b\ Eombg + c\ Eombd + d\ PTAI + e\ Ecuid + f\ Ecuig + g\ PCUI + h\ Emcui + i\ Emomb + Ct,$$

où Emcui est l'épaisseur de graisse moyenne à mi-cuisse, soit la demi-somme de Ecuid et Ecuig, et Emomb est l'épaisseur de graisse moyenne au niveau dorsal, soit la demi-somme de Eombg et Eombd :

- et où a = 0,221, b = 0,032, c = 0,06, d = 0,253, e = 0,078, f = - 0,0128, g = 0,073, h = -0,048, i = -0,028, Ct = -21,2 si la première estimation MG1 est inférieure au seuil Sh égal à 22 kg,
- et a = 1,31, b = -2,69, c = -1,76, d = -0,175, e = 10,33, f = 8,91, g = - 0,123, h = -18,97, i = 4,72, Ct = -10,88 si la première estimation MG1 est supérieure ou égale au seuil Sh égal à 22 kg.

Dans un mode de mise en oeuvre de l'invention, on calcule à l'aide du module 10 la composition corporelle de l'individu. Le poids de l'individu Pds, préalablement stocké dans la mémoire 7 par l'opérateur, est égal à la somme de la masse maigre et de la masse grasse figurant dans son corps. Ainsi, la masse grasse de l'individu ayant été précédemment estimée, la masse maigre MM est obtenue en soustrayant celle-ci au poids de l'individu, soit MM = Pds - MG2.
Puis on estime l'eau corporelle totale Ecorp à l'aide de l'égalité suivante : Ecorp = 0,692 MM + 1,572, où l'eau corporelle totale Ecorp est exprimée en litres et la masse maigre MM en kilogramme.
Dans un mode de réalisation préféré, les valeurs calculées MG2, MM et Ecorp sont enregistrées en mémoire 7. Ceci permet à l'opérateur de pouvoir avoir accès à l'historique relatif à un individu et mettre en évidence l'évolution de son profil au cours du temps.
Dans un autre mode de mise en oeuvre avantageux, l'opérateur utilise une ceinture spéciale 11 à mettre en place avant de réaliser les mesures. Cette ceinture 11 comporte deux repères latéraux dorsaux 12,13 de part et d'autre et à égale distance d'un repère central dorsal 14 et une boucle de fermeture 15 constituant en outre un repère central ombilical. Cette ceinture est à mettre en place autour du tronc de l'individu de sorte que la ceinture soit dans un plan horizontal passant entre les vertèbres L4-L5 et au niveau ombilical. Le repère central dorsal 14 doit être situé sur l'axe vertébral vertical comme représenté sur la figure 3a, et le repère central ombilical 15 doit être situé sur l'ombilic.
Avantageusement deux ceintures différentes peuvent être prévues. Une première est par exemple adaptée à un tour de taille compris entre 50 et 90 cm, et une seconde ceinture convient pour un tour de taille compris entre 80 et 150 cm.

L'opérateur peut ensuite alors placer la sonde sous les repères latéraux dorsaux 12,13 et le repère central ombilical 15.

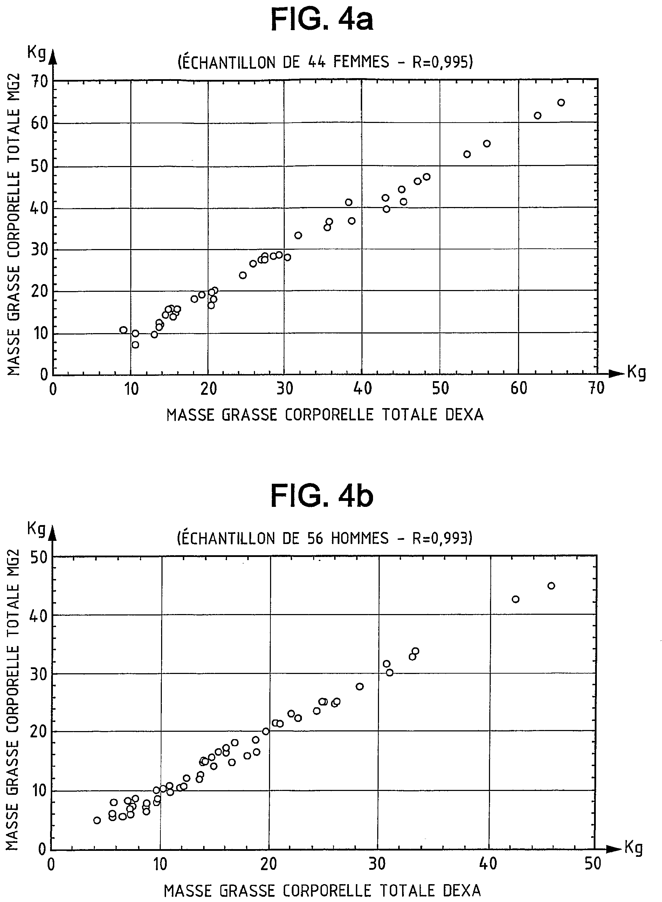

Une détermination de masse grasse corporelle totale selon l'invention est réalisée de façon rapide, aisée. Elle ne nécessite pas d'environnement médical et fournit des résultats fiables. Sur la figure 4a figure un graphe présentant en abscisse la masse grasse MG2 déterminée selon l'invention et en ordonnée la masse grasse fournie par la méthode de référence DEXA pour un échantillon composé de 44 femmes âgées de 18 à 60 ans. Sur la figure 4b figure un graphe présentant en abscisse la masse grasse MG2 déterminée selon l'invention et en ordonnée la masse grasse fournie par la méthode de référence DEXA pour un échantillon composé de 56 hommes âgés de 18 à 60 ans.

Le coefficient de corrélation calculé sur ces échantillons, entre la seconde estimation MG2 obtenue selon l'invention et la valeur de masse grasse corporelle totale fournie par la méthode de référence DEXA est de R = 0,995 pour les femmes (ce qui correspond à une précision de l'ordre de 5%) et de R = 0,993 pour les hommes (soit une précision de l'ordre de 7%).

Le mode de réalisation de l'invention présenté ci-dessus prévoit l'utilisation d'un calculateur 4. Cette configuration est bien adaptée pour effectuer rapidement les différentes déterminations de masse grasse corporelle totale, masse maigre et/ou eau corporelle totale d'un grand nombre d'individus et automatiser l'enrichissement de l'historique.

Ainsi l'invention se prête particulièrement bien au cas où ces déterminations doivent être réalisées de façon portative.

Par ailleurs, l'invention peut s'appliquer à la détermination de la masse grasse corporelle et/ou de la composition corporelle d'individus de profils divers, par exemple des sportifs de haut niveau, des individus présentant une obésité extrême, des enfants, des adolescents etc.

## Revendications

1. Procédé de détermination de la masse grasse corporelle totale d'un individu, comprenant les étapes suivantes:

   - on mesure, à l'aide d'un dispositif à ultrasons, une épaisseur de graisse sous-cutanée en quatre points du corps de l'individu, situés respectivement au niveau antérieur droit de la mi-cuisse gauche, au niveau antérieur gauche de la mi-cuisse droite, au niveau ombilical dorsal droit et au niveau ombilical dorsal gauche;
   - on détermine à l'aide d'un calculateur ensuite une première estimation de la masse grasse corporelle totale de l'individu en fonction d'au moins les mesures d'épaisseur de graisse sous-cutanée en lesdits quatre points,

   la première estimation de la masse grasse corporelle totale de l'individu étant obtenue à l'aide de l'équation ci-dessous: MG = a BMI + b Eombg + c Eombd + d PTAI + e Ecuid + f Ecuig + g PCUI + Ct, où MG est la première estimation de la masse grasse corporelle totale en kg, BMI est le rapport entre le poids en kg et le carré de la stature en m, PTAI est le périmètre du tour de taille passant par l'ombilic, PCUI est la moyenne des périmètres du tour de jambes gauche et droite passant à mi-cuisse, Eombg, Eombd, Ecuid et Ecuig sont les épaisseurs de graisse mesurées respectivement au niveau ombilical dorsal gauche, au niveau ombilical dorsal droit, au niveau antérieur droit de la mi-cuisse gauche et au niveau antérieur gauche de la mi-cuisse droite, en mm, et où a = 0,959, b = 0,119, c = -0,044, d = 0,13, e = 0,586, f = -0,329, g =0,061, Ct -23, 88, lorsque l'individu est de sexe féminin, et a = 0,733, b = -0,053, c = 0,102, d = 0,27, e = 0,155, f = -0,106, g = 0,015, Ct = -31,21, lorsque l'individu est de sexe masculin.

2. Procédé selon la revendication 1, selon lequel la première estimation de la masse grasse corporelle totale de l'individu est en outre fonction de valeurs représentatives du périmètre du tour de taille passant par l'ombilic, du périmètre du tour de jambe passant à mi-cuisse, de l'indice de corpulence défini comme le rapport entre le poids et le carré de la stature, et du sexe de l'individu.

3. Procédé selon la revendication 1 ou la revendication 2, selon lequel la première estimation de la masse grasse corporelle totale de l'individu est une fonction linéaire des mesures d'épaisseur de graisse sous-cutanée en lesdits quatre points.

4. Procédé selon l'une quelconque des revendications précédentes, selon lequel:

   - on compare la première estimation de la masse grasse corporelle totale de l'individu à un seuil défini par sexe,
   - et on détermine ensuite une seconde estimation de la masse grasse corporelle totale de l'individu en fonction des résultats de la comparaison et d'au moins des mesures d'épaisseur de graisse sous-cutanée en lesdits quatre points.

**5.** Procédé selon la revendication 4, selon lequel la seconde estimation de la masse grasse corporelle totale de l'individu est en outre fonction au moins de valeurs représentatives du périmètre du tour de taille passant par l'ombilic, du périmètre du tour de jambe passant à mi-cuisse, de l'indice de corpulence défini comme le rapport entre le poids et le carré de la stature, et du sexe de l'individu.

**6.** Procédé selon la revendication 4 ou la revendication 5, selon lequel la seconde estimation de la masse grasse corporelle totale de l'individu est obtenue à l'aide d'une fonction linéaire des mesures d'épaisseur cutanée en lesdits quatre points.

**7.** Procédé selon l'une des revendications 4 à 6, selon lequel la seconde estimation de la masse grasse corporelle totale de l'individu est obtenue à l'aide de l'équation ci-dessous: MG = a BMI + b Eombg + c Eombd + d PTAI + e Ecuid + f Ecuig + g PCUI + Ct, lorsque l'individu est de sexe féminin où a = 0,947, b = 0,079, c = 0,0067, d = 0,103, e = -0,303, f = 0, 379, g =0,072, Ct = - 20,58 si la première estimation est inférieure au seuil prédéfini de 42 kg, et a = -0,743, b = - 1,73, c = 1,10, d = 1,677, e = -3,35, f = 2,62, g = -4,95, Ct = 242,53 si la première estimation est supérieure ou égale au seuil prédéfini de 42 kg, et à l'aide de l'équation suivante lorsque l'individu est de sexe masculin: MG = a BMI + b Eombg + c Eombd + d PTAI + e Ecuid + f Ecuig + g PCUI + h Emcui + i Emomb + Ct, où Emcui est l'épaisseur de graisse moyenne à mi-cuisse et Emomb est l'épaisseur de graisse moyenne au niveau dorsal et où a = 0,221, b = 0,032, c = 0,06, d = 0,253, e = 0,078, f = -0,0128, g = 0,073, h = 0,048, i = -0,028, Ct = -21,2 si la première estimation est inférieure au seuil prédéfini de 22 kg, et a = 1,31, b = -2,69, c = -1,76, d = -0,175, e = 10,33, f = 8,91, g = -0,123, h = -18,97, i = 4,72, Ct = -10,88 si la première estimation est supérieure ou égale au seuil prédéfini de 22 kg.

**8.** Procédé de détermination de la composition corporelle d'un individu selon lequel:

- on réalise les étapes d'un procédé de détermination de la masse grasse corporelle totale de individu selon l'une quelconque des revendications précédentes;
- à partir de la masse grasse déterminée, on détermine une valeur de masse maigre et une valeur d'eau corporelle totale présentes dans le corps de l'individu.

**9.** Procédé selon la revendication 8, selon lequel la valeur de la masse maigre est égale au poids de l'individu auquel on soustrait une masse grasse déterminée et la valeur d'eau corporelle totale est égale à : Ecorp = 0,692 MM + 1,572, où Ecorp est l'eau corporelle totale (en litre) et MM la masse maigre (en kilogramme).

**10.** Système de détermination de la masse grasse corporelle totale d'un individu, comprenant:

- un dispositif à ultrasons (2) ;
- un module de détermination d'épaisseur de masse grasse sous-cutanée (6) configuré pour traiter les données issues du dispositif à ultrasons (2) de manière à en extraire des valeurs représentatives d'une épaisseur de graisse sous-cutanée en quatre points du corps de l'individu, situés respectivement au niveau antérieur droit de la mi-cuisse gauche, au niveau antérieur gauche de la mi-cuisse droite, au niveau ombilical dorsal droit et au niveau ombilical dorsal gauche;
- un module de détermination de masse grasse corporelle totale (8) configuré pour déterminer une première estimation de la masse grasse corporelle totale de l'individu en fonction d'au moins les mesures d'épaisseur de graisse sous-cutanée en lesdits quatre points, la première estimation de la masse grasse corporelle totale de l'individu étant obtenue à l'aide de l'équation ci-dessous: MG = a BMI + b Eombg + c Eombd + d PTAI + e Ecuid + f Ecuig + g PCUI + Ct, où MG est la première estimation de la masse grasse corporelle totale en kg, BMI est le rapport entre le poids en kg et le carré de la stature en m, PTAI est le périmètre du tour de taille passant par l'ombilic, PCUI est la moyenne des périmètres du tour de jambes gauche et droite passant à mi-cuisse, Eombg, Eombd, Ecuid et Ecuig sont les épaisseurs de graisse mesurées respectivement au niveau ombilical dorsal gauche, au niveau ombilical dorsal droit, au niveau antérieur droit de la mi-cuisse gauche et au niveau antérieur gauche de la mi-cuisse droite, en mm, et où a = 0,959, b = 0,119, c = -0,044, d = 0,13, e = 0,586, f = -0,329, g =0,061, Ct = -23, 88, lorsque l'individu est de sexe féminin, et a = 0,733, b = -0,053, c = 0,102, d = 0,27, e = 0,155, f = -0,106, g = 0,015, Ct = -31,21, lorsque l'individu est de sexe masculin.

**11.** Système de détermination de la composition corporelle d'un individu, comprenant:

- un système de détermination de la masse grasse corporelle totale d'un individu selon la revendication 10,
- un module de détermination de composition corporelle totale (10) configuré pour déterminer, à partir de la masse grasse déterminée, une valeur de masse maigre et une valeur d'eau corporelle totale présentes dans le corps de l'individu.

**Patentansprüche**

1. Verfahren zur Bestimmung der Gesamtmenge an Körperfett einer Person, das die folgenden Schritte umfasst:

   - Messen einer Dicke des subkutanen Fetts an vier Punkten des Körpers der Person, die sich im rechten vorderen Bereich der Mitte des linken Oberschenkels, im linken vorderen Bereich der Mitte des rechten Oberschenkels, im rechten dorsalen Nabelbereich beziehungsweise im linken dorsalen Nabelbereich befinden, mittels einer Ultraschallvorrichtung;
   - anschließend Bestimmen einer ersten Schätzung der Gesamtmenge an Körperfett der Person in Abhängigkeit von mindestens den Messungen der Dicke des subkutanen Fetts an den vier Punkten mittels eines Rechners,

   wobei die erste Schätzung der Gesamtmenge an Körperfett der Person mittels der folgenden Gleichung erhalten wird: MG = a BMI + b Eombg + c Eombd + d PTAI + e Ecuid + f Ecuig + g PCUI + Ct, wo MG die erste Schätzung der Gesamtmenge an Körperfett in kg ist, BMI das Verhältnis zwischen dem Gewicht in kg und dem Quadrat der Körperlänge in m ist, PTAI der Taillenumfang ist, der über den Bauchnabel verläuft, PCUI das Mittel der Umfänge des linken und des rechten Beins ist, die über die Mitte des Oberschenkels verlaufen, Eombg, Eombd, Ecuid und Ecuig die im linken dorsalen Nabelbereich, im rechten dorsalen Nabelbereich, im rechten vorderen Bereich der Mitte des linken Oberschenkels beziehungsweise im linken vorderen Bereich der Mitte des rechten Oberschenkels gemessenen Fettdicken in mm sind und wo a = 0,959, b = 0,119, c = -0,044, d = 0,13, e = 0,586, f = -0,329, g = 0,061, Ct = -23,88, wenn die Person weiblichen Geschlechts ist, und a = 0,733, b = -0,053, c = 0,102, d = 0,27, e = 0,155, f = -0,106, g = 0,015, Ct = -31,21 ist, wenn die Person männlichen Geschlechts ist.

2. Verfahren nach Anspruch 1, wobei die erste Schätzung der Gesamtmenge an Körperfett der Person ferner abhängig von Werten ist, die für den Taillenumfang, der über den Bauchnabel verläuft, den Beinumfang, der über den mittleren Oberschenkel verläuft, den als das Verhältnis zwischen dem Gewicht und der Körperlänge im Quadrat definierten Body-Mass-Index und für das Geschlecht der Person charakteristisch sind.

3. Verfahren nach Anspruch 1 oder Anspruch 2, wobei die erste Schätzung der Gesamtmenge an Körperfett der Person eine lineare Funktion der Messungen der Dicke des subkutanen Fetts an den vier Punkten ist.

4. Verfahren nach einem der vorhergehenden Ansprüche, wobei:

   - die erste Schätzung der Gesamtmenge an Körperfett der Person mit einem für jedes Geschlecht definierten Schwellenwert verglichen wird,
   - und dann eine zweite Schätzung der Gesamtmenge an Körperfett der Person in Abhängigkeit von den Ergebnissen des Vergleichs von mindestens den Messungen der Dicke des subkutanen Fetts an den vier Punkten bestimmt wird.

5. Verfahren nach Anspruch 4, wobei die zweite Schätzung der Gesamtmenge an Körperfett der Person ferner mindestens von Werten abhängig ist, die für den Taillenumfang, der über den Bauchnabel verläuft, den Beinumfang, der über die Mitte des Oberschenkels verläuft, den als das Verhältnis zwischen dem Gewicht und der Körpergröße im Quadrat definierten Body-Mass-Index und das Geschlecht der Person charakteristisch sind.

6. Verfahren nach Anspruch 4 oder Anspruch 5, wobei die zweite Schätzung der Gesamtmenge an Körperfett der Person mittels einer linearen Funktion der Messungen der kutanen Dicke an den vier Punkten erhalten wird.

7. Verfahren nach einem der Ansprüche 4 bis 6, wobei die zweite Schätzung der Gesamtmenge an Körperfett der Person mittels der folgenden Gleichung erhalten wird: MG = a BMI + b Eombg + c Eombd + d PTAI + e Ecuid + f Ecuig + g PCUI + Ct, wenn die Person weiblichen Geschlechts ist, wo a = 0,947, b = 0,079, c = 0,0067, d = 0,103, e = -0,303, f = 0,379, g = 0,072, Ct = -20,58, wenn die erste Schätzung niedriger als der vordefinierte Schwellenwert von 42 kg ist, und a = -0,743, b = -1,73, c = 1,10, d = 1,677, e = -3,35, f = 2,62, g = -4,95, Ct = 242,53, wenn die erste Schätzung höher oder gleich dem vordefinierten Schwellenwert von 42 kg ist, und mittels der folgenden Gleichung, wenn die Person männlichen Geschlechts ist: MG = a BMI + b Eombg + c Eombd + d PTAI + e Ecuid + f Ecuig + g PCUI + h Emcui + i Emomb + Ct, wo Emcui die mittlere Fettdicke am mittleren Oberschenkel ist und Emomb die mittlere Fettdicke im dorsalen Bereich ist und wo a = 0,221, b = 0,032, c = 0,06, d = 0,253, e = 0,078, f = -0,0128, g = 0,073, h = 0,048, i = -0,028, Ct = -21,2, wenn die erste Schätzung niedriger als der vordefinierte Schwellenwert von 22 kg ist, und a = 1,31, b = -2,69, c = -1, 76, d = -0,175, e = 10,33, f = 8, 91, g = -0,123, h = -18,97, i = 4,72, Ct = -10,88, wenn die erste Schätzung höher als oder gleich dem vordefinierten Schwellenwert von 22 kg ist.

8. Verfahren zur Bestimmung der Gesamtmenge an Körperfett einer Person: wobei:

- die Schritte eines Verfahrens zur Bestimmung der Gesamtmenge an Körperfett der Person nach einem der vorhergehenden Ansprüche durchgeführt werden;
- ausgehend von der bestimmten Menge an Fett ein Wert der Magermasse und ein Gesamtwert an Körperwasser, die im Körper der Person vorhanden sind, bestimmt werden.

9. Verfahren nach Anspruch 8, wobei der Wert der Magermasse gleich dem Gewicht der Person ist, von dem eine bestimmte Körperfettmenge abgezogen wurde, und der Gesamtwert an Körperwasser gleich Folgendem ist: Ecorp = 0,692 MM + 1,572, wo Ecorp die Gesamtmenge an Körperwasser (in Liter) und MM die Magermasse (in Kilogramm) ist.

10. System zur Bestimmung der Gesamtmenge an Körperfett einer Person, das Folgendes umfasst:

- eine Ultraschallvorrichtung (2);
- ein Modul zur Bestimmung der Dicke der Menge an subkutanem Fett (6), das ausgestaltet ist, um die Daten, die von der Ultraschallvorrichtung (2) stammen, derart zu verarbeiten, dass davon Werte gewonnen werden, die für eine Dicke des subkutanen Fetts an vier Punkten des Körpers der Person charakteristisch sind, die sich im rechten vorderen Bereich der Mitte des linken Oberschenkels, im linken vorderen Bereich der Mitte des rechten Oberschenkels, im rechten dorsalen Nabelbereich beziehungsweise im linken dorsalen Nabelbereich befinden;
- ein Modul zur Bestimmung der Gesamtmenge an Körperfett (8), das ausgestaltet ist, um eine erste Schätzung der Gesamtmenge an Körperfett der Person in Abhängigkeit von mindestens den Messungen der Dicke des subkutanen Fetts an den vier Punkten zu bestimmen, wobei die erste Schätzung der Gesamtmenge an Körperfett der Person mittels der folgenden Gleichung erhalten wird: MG = a BMI + b Eombg + c Eombd + d PTAI + e Ecuid + f Ecuig + g PCUI + Ct, wo MG die erste Schätzung der Gesamtmenge an Körperfett in kg ist, BMI das Verhältnis zwischen dem Gewicht in kg und dem Quadrat der Körperlänge in m ist, PTAI der Taillenumfang ist, der über den Bauchnabel verläuft, PCUI das Mittel der Umfänge des linken und des rechten Beins ist, die über die Mitte des Oberschenkels verlaufen, Eombg, Eombd, Ecuid und Ecuig die im linken dorsalen Nabelbereich, im rechten dorsalen Nabelbereich im rechten vorderen Bereich der Mitte des linken Oberschenkels beziehungsweise im linken vorderen Bereich der Mitte des rechten Oberschenkels gemessenen Fettdicken in mm sind und wo a = 0,959, b = 0,119, c = -0,044, d = 0,13, e = 0,586, f = -0,329, g = 0,061, Ct = -23,88, wenn die Person weiblichen Geschlechts ist, und a = 0,733, b = -0,053, c = 0,102, d = 0,27, e = 0,155, f = -0,106, g = 0,015, Ct = -31,21 ist, wenn die Person männlichen Geschlechts ist.

11. System zur Bestimmung der Körperzusammensetzung einer Person, das Folgendes umfasst:

- ein System zur Bestimmung der Gesamtmenge an Körperfett einer Person nach Anspruch 10,
- ein Modul zur Bestimmung der Gesamtkörperzusammensetzung (10), das ausgestaltet ist, um ausgehend von der bestimmten Menge an Fett einen Wert an Magermasse und einen Gesamtwert an Körperwasser zu bestimmen, die in dem Körper der Person vorhanden sind.

**Claims**

1. Method of determining the total body fat of a person, comprising the following steps:

- measuring, using an ultrasound device, a subcutaneous fat thickness at four points on the person's body, located in the right anterior part of the left mid-thigh, in the left anterior part of the right mid-thigh, in the right dorsal part at navel level and in the left dorsal part at navel level; and then
- determining, using a computer, a first estimate of the person's total body fat as a function of at least the subcutaneous fat thickness measurements at said four points,

the first estimate of the person's total body fat being obtained using the equation below: FAT=$a$×BMI+$b$×$T_{nav,l}$+c×$T_{nav,r}$+$d$×WP+$e$×$T_{thigh,r}$+$f$×$T_{thigh,l}$+$g$×$AP_{thigh}$ +constant, where FAT is the first estimate of the total body fat in kg, BMI is the ratio of the person's weight in kg to the square of his height in m, WP is the waist perimeter passing through the navel, $AP_{thigh}$ is the average of the left and right leg perimeters passing at mid-thigh, $T_{nav,l}$, $T_{nav,r}$, $T_{thigh,r}$ and $T_{thigh,l}$ are the fat thicknesses measured in the left dorsal part at navel level, in the right dorsal part at navel level, in the right anterior part of the left mid-thigh and in the left anterior part of the right mid-thigh, in mm, respectively, and where a=0.959, b=0.119, c=-0.044, d=0.13, e=0.586, f=-0.329, g=0.061 and constant=-23.88, when the person is of the female sex; and a=0.733, b=-0.053, c=0.102, d=0.27, e=0.155, f=-0.106, g=0.015 and constant=-31.21 when the person is of the male sex.

2.  The method as claimed in claim 1, wherein the first estimate of the person's total body fat is also a function of values representative of the perimeter around the waist passing through the navel, the perimeter around the leg passing through mid-thigh, the body mass index, defined as the ratio of a person's weight to the square of his height, and the sex of the person.

3.  The method as claimed in claim 1 or claim 2, wherein the first estimate of the person's total body fat is a linear function of the subcutaneous fat thickness measurements at said four points

4.  The method as claimed in any one of the preceding claims, wherein:

    - the first estimate of the person's total body fat is compared with a threshold defined by sex, and
    - a second estimate of the person's total body fat is then determined as a function of the results of the comparison and of at least the subcutaneous fat thickness measurements at said four points.

5.  The method as claimed in claim 4, wherein the second estimate of the person's total body fat is also a function of at least values representative of the perimeter around the waist passing through the navel, the perimeter around the leg passing through mid-thigh, the body mass index, defined as the ratio of the person's weight to the square of his height, and the sex of the person.

6.  The method as claimed in claim 4 or claim 5, wherein the second estimate of the person's total body fat is obtained using a linear function of the subcutaneous fat thickness measurements at said four points.

7.  The method as claimed in one of claims 4 to 6, wherein the second estimate of the person's total body fat is obtained using the equation below: FAT=$a$×BMI+$b$×$T_{nav,l}$+c×$T_{nav,r}$+$d$×WP+$e$×$T_{thigh,r}$+$f$×$T_{thigh,l}$+$g$×$AP_{thigh}$ +constant, when the person is of the female sex, where a=0.947, b=0.079, c=0.0067, d=0.103, e=-0.303, f=0.379, g=0.072 and constant -20.58 if the first estimate is below the predefined threshold of 42 kg, and a=-0.743, b=-1.73, c=1.10, d=1.677, e=-3.35, f=2.62, g=-4.95 and constant 242.53 if the first estimate is equal to or greater than the predefined threshold of 42 kg, and using the following equation when the person is of the male sex: FAT=$a$×BMI+$b$×$T_{nav,l}$+c×$T_{nav,r}$+$d$×WP+$e$×$T_{thigh,r}$+$f$×$T_{thigh,l}$+$g$×$A$ $P_{thigh}$+$h$×$T_{thigh,av}$+$i$×$T_{nav,av}$+constant, where $T_{thigh,av}$ is the average fat thickness at mid-thigh and $T_{nav,av}$ is the average dorsal fat thickness at navel level, and where a=0.221, b=0.032, c=0.06, d=0.253, e=0.078, f=-0.0128, g=0.073, h=0.048, i=-0.028 and constant=-21.2 if the first estimate is below the predefined threshold of 22 kg, and a=1.31, b=-2.69, c=-1.76, d=-0.175, e=10.33, f=8.91, g=-0.123, h=-18.97, i=4.72 and constant -10.88 if the first estimate is equal to or greater than the predefined threshold of 22 kg.

8.  A method of determining the body composition of a person, comprising:

    - carrying out the steps of a method of determining the total body fat of a person as claimed in any one of the preceding claims;
    - from the body fat determined, determining a value of the lean body mass and a value of the total body water present in the person's body.

9.  The method as claimed in claim 8, wherein the value of the lean body mass is equal to the person's weight from which a determined fat value is subtracted and the total body water value is equal to: $W_{body}$ =0.692×LBM+1.572, where $W_{body}$ is the total body water (in liters) and LBM is the lean body mass (in kilograms).

10. A system for determining the total body fat of a person, comprising:

- an ultrasound device (2);
- a subcutaneous fat thickness determination module (6) configured for processing the data from the ultrasound device (2) so as to extract therefrom representative values of a subcutaneous fat thickness at four points on the person's body, located in the right anterior part of the left mid-thigh, in the left anterior part of the right mid-thigh, in the right dorsal part at navel level and in the left dorsal part at navel level, respectively;
- a total body fat determination module (8) configured for determining a first estimate of the person's total body fat as a function of at least the subcutaneous fat thickness measurements at said four points, the first estimate of the person's total body fat being obtained using the equation below: $FAT = a \times BMI + b \times T_{nav,l} + c \times T_{nav,r} + d \times WP + e \times T_{thigh,r} + f \times T_{thigh,l} + g \times AP_{thigh}$ +constant, where FAT is the first estimate of the total body fat in kg, BMI is the ratio of the person's weight in kg to the square of his height in m, WP is the waist perimeter passing through the navel, $AP_{thigh}$ is the average of the left and right leg perimeters passing at mid-thigh, $T_{nav,l}$, $T_{nav,r}$, $T_{thigh,r}$ and $T_{thigh,l}$ are the fat thicknesses measured in the left dorsal part at navel level, in the right dorsal part at navel level, in the right anterior part of the left mid-thigh and in the left anterior part of the right mid-thigh, in mm, respectively, and where a=0.959, b=0.119, c=-0.044, d=0.13, e=0.586, f=-0.329, g=0.061 and constant=-23.88, when the person is of the female sex; and a=0.733, b=-0.053, c=0.102, d=0.27, e=0.155, f=-0.106, g=0.015 and constant=-31.21 when the person is of the male sex.

11. A system for determining the body composition of a person, comprising:

- a system for determining the total body fat of a person as claimed in claim 10,
- a total body composition determination module (10) configured for determining, from the body fat determined, a value of the lean body mass and a value of the total body water present in the person's body.

FIG. 1

EP 1 680 025 B1

# FIG. 2a

# FIG. 2b

# FIG. 3a

# FIG. 3b

# FIG. 4a

(ÉCHANTILLON DE 44 FEMMES – R=0,995)

MASSE GRASSE CORPORELLE TOTALE MG2 (Kg) vs MASSE GRASSE CORPORELLE TOTALE DEXA (Kg)

# FIG. 4b

(ÉCHANTILLON DE 56 HOMMES – R=0,993)

MASSE GRASSE CORPORELLE TOTALE MG2 (Kg) vs MASSE GRASSE CORPORELLE TOTALE DEXA (Kg)

## RÉFÉRENCES CITÉES DANS LA DESCRIPTION

**Documents brevets cités dans la description**

- US 5941825 A
- EP 1055396 A
- WO 9965395 A

**Littérature non-brevet citée dans la description**

- **HC LUKASKI.** Methods for the assessment of human body composition : traditional and new. *Am J clin Nutr,* 1987, vol. 46, 537-56
- **HAN TS ; KELLY IE ; WALSH K ; GREENE RME ; LEAN MEJ.** Relationship between volumes and areas from single transverse scans of intra abdominal fat measured by magnetic resonance imaging. *Int J Obes,* 1997, vol. 21, 1161-1166
- **FRIEDL K E ; WESTPHAL K A ; MARCHITELLI L J ; PATTON J F ; CAMERON CHUMLEA W ; GUO S S.** Evaluation of anthorpometric equations to assess body-composition changes in young women. *Ann. J. Clin. Nutr.,* 2001, vol. 73, 268-275